# EUROPEAN PATENT APPLICATION

(11) **EP 4 814 004 A1**
(43) Date of publication of application: **30.09.2026**
(21) Application number: 24892588.5
(22) Date of filing: 25.01.2024
(51) Int. Cl.: C07K 14/705, A61K 39/00, A61P 35/00

(54) **PD-1 MOLECULE FOR BLOCKING BINDING OF ANTI-PD-1 ANTIBODY TO PD-1 MOLECULE ON CELL SURFACE, AND MUTANT AND USE THEREOF**

(30) Priority: 24.11.2023 CN 202311586651
(71) Applicant: Shenzhen Pregene Biopharma Co., Ltd., Shenzhen, Guangdong 518000 (CN)
(72) Inventor: GAO, Quanli, Zhengzhou, Henan 450008 (CN); YOU, Hongqin, Zhengzhou, Henan 450008 (CN); CHEN, Guangyu, Zhengzhou, Henan 450008 (CN)
(74) Representative: Murgitroyd & Company
(86) International application number: PCT/CN2024/074060
(87) International publication number: WO 2025/107419

(57) **Abstract**

Provided are a PD-1 molecule for blocking the binding of an anti-PD-1 antibody to a PD-1 molecule on a cell surface, and a mutant and the use thereof. Further provided is a protein molecule, which has 80-100% sequence identity to a wild-type protein as shown in SEQ ID NO: 1. The provided recombinant protein H126 and H134 molecules can competitively bind to an anti-PD-1 antibody drug with the endogenous PD-1 molecule expressed by a T cell, without affecting the binding of PD-1 to an endogenous PD-L1 molecule, thereby restoring the function suppression on activated T cells and alleviating the symptoms of immunotherapy-related adverse events. By means of the protein molecule, a new way of performing clinical treatment of the immunotherapy-related adverse events is provided.

## Description

The present disclosure claims priority to Chinese Patent Application No. 2023115866519, filed with the China National Intellectual Property Administration on November 24, 2023, entitled "PD-1 MOLECULE FOR BLOCKING BINDING OF ANTI-PD-1 ANTIBODY TO PD-1 MOLECULE ON CELL SURFACE, AND MUTANT AND USE THEREOF", which is incorporated herein by reference in its entirety.

### TECHNICAL FIELD

The present invention belongs to the field of biomedicine, and specifically relates to a group of PD-1 molecules for blocking the binding of an anti-PD-1 antibody to a PD-1 molecule on a cell surface, and the mutants and the uses thereof.

### BACKGROUND ART

PD-1 is an immunosuppressive molecule expressed on T cells. When anti-PD-1 antibodies bind to PD-1, they block the binding of PD-1 to its ligand PD-L1, thereby relieving the inhibitory signal of PD-1 and restoring the tumor-killing function of T cells. Anti-PD-1 antibodies are currently the main immunotherapy for malignant tumors, but more than half of patients experience immune-related adverse events (irAEs) after treatment, more than 20% of patients need to discontinue treatment, and nearly 10% of patients need treatment with glucocorticoids (GCs). How to control irAEs caused by anti-PD-1 therapy is one of the most pressing problems to be solved in current tumor immunotherapy.

Although GCs are the primary and effective treatment for irAEs, some patients still develop resistance to the treatment, and long-term use of GCs has significant side effects for patients with malignant tumors. Currently, several drugs for the treatment of irAEs are available in the art. For example, Chinese Patent Application No. 202310565962.0 discloses a CD80 and CD86 binding protein composition and use thereof, which relates to a cytotoxic T-lymphocyte-associated antigen-4 (CTLA-4) protein composition and its use in alleviating autoimmune adverse events associated with cancer immunotherapy. U.S. Patent Application 202017625226 discloses a method for detecting follicular regulatory T cells (TFR), the method comprising: obtaining a biological sample from a subject and contacting the biological sample with an antibody that detects CD3+CD4+FOXP3+BCL6+ T cells, CD3+CD4+CXCR5+GITR+ T cells, or both, and detecting an increase in TFR in the tumor sample. The invention further includes combination therapy that depletes follicular regulatory T lymphocytes (TFR) in a manner that minimizes the impact on regulatory T cells (TREGS) to reduce immune-related adverse events (irAEs).

Therefore, there is a need in the art for additional drugs for the treatment of irAEs.

### SUMMARY OF THE INVENTION

The trigger for irAEs is anti-PD-1 drug therapy. When the therapeutic drug is unable to bind to the endogenous PD-1 expressed by T cells, this trigger disappears. Therefore, the present invention designs a molecule that can block the binding of anti-PD-1 antibody drugs to the endogenous PD-1 expressed by T cells, in order to treat patients with irAEs.

To overcome the shortcomings of the prior art, the present invention proposes the following technical solution:
In one aspect, the present invention provides a protein molecule that has 80%-100% sequence identity with a wild-type protein as shown in SEQ ID NO: 1.

In some embodiments, the protein molecule is a functional variant that differs from the wild-type protein as shown in SEQ ID NO: 1 by at least one amino acid position;
the difference at amino acid positions is achieved by at least one of addition, deletion, modification and/or substitution of amino acids;
the functional variant has the same, similar, or better function in blocking the binding of anti-PD-1 antibodies to PD-1 molecules as the wild-type protein as shown in SEQ ID NO: 1.

In some preferred embodiments, the difference at amino acid positions is achieved by substitution of amino acids, preferably by conservative substitution of amino acids.

In some embodiments, the protein molecule comprises an amino acid substitution at position 126 or 134 compared to the wild-type protein as shown in SEQ ID NO: 1.

In some preferred embodiments, the protein molecule comprises an amino acid substitution of I126A compared to the wild-type protein as shown in SEQ ID NO: 1.

In some preferred embodiments, the protein molecule has the amino acid sequence as shown in SEQ ID NO: 2.

In some embodiments, the protein molecule comprises an amino acid substitution of I134A compared to the wild-type protein as shown in SEQ ID NO: 1.

In some preferred embodiments, the protein molecule has the amino acid sequence as shown in SEQ ID NO: 3.
SEQ ID NO: 1: 
MQIPQAPWPVVWAVLQLGWRPGWFLDSPDRPWNPPTFSPALLVVTEGDNATFTCSFSN
TSESFVLNWYRMSPSNQTDKLAAFPEDRSQPGQDCRFRVTQLPNGRDFHMSVVRARRN
DSGTYLCGAISLAPKAQIKESLRAELRVTERRAEVPTAHPSPSPRPAGQFQTLV.
SEQ ID NO: 2:

MQIPQAPWPVVWAVLQLGWRPGWFLDSPDRPWNPPTFSPALLVVTEGDNATFTCSFSN
TSESFVLNWYRMSPSNQTDKLAAFPEDRSQPGQDCRFRVTQLPNGRDFHMSVVRARRN
DSGTYLCGAASLAPKAQIKESLRAELRVTERRAEVPTAHPSPSPRPAGQFQTLV.

The nucleotide sequence encoding SEQ ID NO: 2 is shown in SEQ ID NO: 4:

ATGCAGATCCCCCAGGCTCCTTGGCCTGTGGTGTGGGCTGTGCTGCAGCTGGGATGG
AGGCCTGGATGGTTCCTGGACTCCCCTGACCGTCCTTGGAACCCTCCTACCTTCTCCC
CCGCTCTCCTCGTTGTCACCGAGGGAGATAACGCCACCTTCACCTGCTCCTTCTCCAA
CACCTCCGAGTCCTTCGTCCTCAACTGGTATCGCATGTCTCCCTCCAACCAAACCGAC
AAGCTGGCCGCTTTCCCCGAAGATAGAAGCCAACCCGGCCAGGACTGCAGATTCCG
TGTGACACAGCTGCCCAACGGGCGAGATTTCCACATGAGCGTCGTGAGGGCCAGAA
GAAACGACTCCGGGACCTACCTGTGTGGCGCAGCTTCTCTGGCTCCCAAGGCTCAAA
TCAAAGAAAGCCTCCGGGCCGAGCTGAGAGTGACTGAAAGAAGAGCTGAGGTGCCC
ACCGCCCACCCTTCTCCTTCTCCTAGACCCGCTGGACAGTTCCAAACCCTCGTGTGA.
SEQ ID NO: 3:

MQIPQAPWPVVWAVLQLGWRPGWFLDSPDRPWNPPTFSPALLVVTEGDNATFTCSFSN
TSESFVLNWYRMSPSNQTDKLAAFPEDRSQPGQDCRFRVTQLPNGRDFHMSVVRARRN
DSGTYLCGAISLAPKAQAKESLRAELRVTERRAEVPTAHPSPSPRPAGQFQTLV.

The nucleotide sequence encoding SEQ ID NO: 3 is shown in SEQ ID NO: 5: 
ATGCAGATCCCCCAGGCTCCTTGGCCTGTGGTGTGGGCTGTGCTGCAGCTGGGATGG
AGGCCTGGATGGTTCCTGGACTCCCCTGACCGTCCTTGGAACCCTCCTACCTTCTCCC
CCGCTCTCCTCGTTGTCACCGAGGGAGATAACGCCACCTTCACCTGCTCCTTCTCCAA
CACCTCCGAGTCCTTCGTCCTCAACTGGTATCGCATGTCTCCCTCCAACCAAACCGAC
AAGCTGGCCGCTTTCCCCGAAGATAGAAGCCAACCCGGCCAGGACTGCAGATTCCG
TGTGACACAGCTGCCCAACGGGCGAGATTTCCACATGAGCGTCGTGAGGGCCAGAA
GAAACGACTCCGGGACCTACCTGTGTGGCGCAATCTCCCTGGCTCCAAAAGCCCAAG
CCAAAGAAAGCCTCCGGGCCGAACTGAGAGTGACCGAAAGAAGAGCCGAGGTGCC
CACCGCACACCCTTCTCCTTCACCTCGACCTGCTGGACAGTTCCAAACCCTCGTGTGA
 .

In another aspect, the present invention provides a recombinant protein comprising any one of the aforementioned protein molecules.

In some embodiments, the recombinant protein further comprises a bioactive protein or a functional fragment thereof that assists in its expression and/or secretion, or prolongs its half-life in vivo.

In some embodiments, the bioactive polypeptide or functional fragment thereof is selected from at least one of immunoglobulin Fc domain, serum albumin, albumin-binding polypeptide, prealbumin, carboxyl-terminal peptide, elastin-like polypeptide, His tag, GST tag, MBP tag, FLAG tag, and SUMO tag.

In another aspect, the present invention provides an anti-PD-1 antibody blocker that competitively binds to the anti-PD-1 antibody with endogenous PD-1, does not bind to endogenous PD-L1, and does not block the binding of endogenous PD-L1 to endogenous PD-1, wherein the blocker comprises any one of the aforementioned protein molecules or any one of the aforementioned recombinant proteins.

In some embodiments, the blocker is formulated with a buffer.

In some embodiments, the anti-PD-1 antibody includes, but is not limited to, pembrolizumab, nivolumab, sintilimab, tislelizumab, and toripalimab.

In another aspect, the present invention provides the use of any one of the aforementioned protein molecules, any one of the aforementioned recombinant proteins, or any one of the aforementioned blockers in the preparation of drugs for treating immune-related adverse events (irAEs).

In some embodiments, the immune-related adverse events are caused by anti-PD-1 antibodies.

In yet another aspect, the present invention provides the use of a protein molecule in the preparation of a drug for treating immune-related adverse events, wherein the protein molecule has the amino acid sequence as shown in SEQ ID NO: 1.

The H126 recombinant protein and H134 recombinant protein provided by the present invention can competitively bind to anti-PD-1 antibody drugs with the endogenous PD-1 molecules expressed on T cells, without affecting the binding of PD-1 to endogenous PD-L1 molecules, thereby restoring the function suppression on activated T cells and alleviating the symptoms of immune-related adverse events. It provides a novel approach for the clinical treatment of immune-related adverse events.

### BRIEF DESCRIPTION OF DRAWINGS

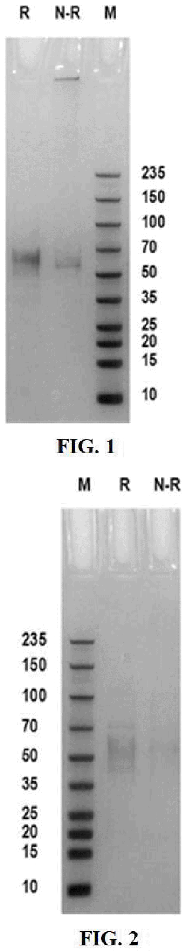

FIG. 1 shows the SDS-PAGE results of H126 recombinant protein, where M represents the protein marker, R represents the reduced H126 recombinant protein, N-R represents the non-reduced H126 recombinant protein, and the unit of the marker is KD.
FIG. 2 shows the SDS-PAGE results of H134 recombinant protein, where M represents the protein marker, R represents the reduced H134 recombinant protein, N-R represents the non-reduced H134 recombinant protein, and the unit of the marker is KD.

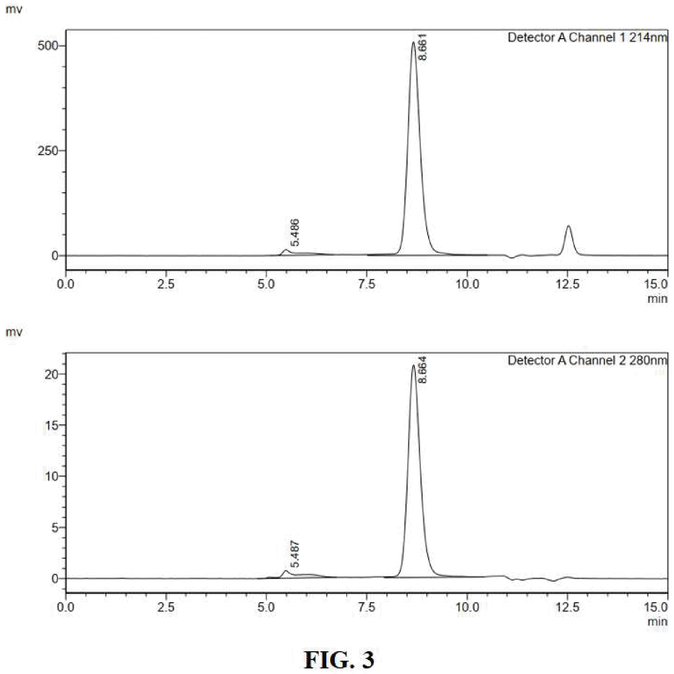

FIG. 3 shows the SEC-HPLC analysis results of H126 recombinant protein.

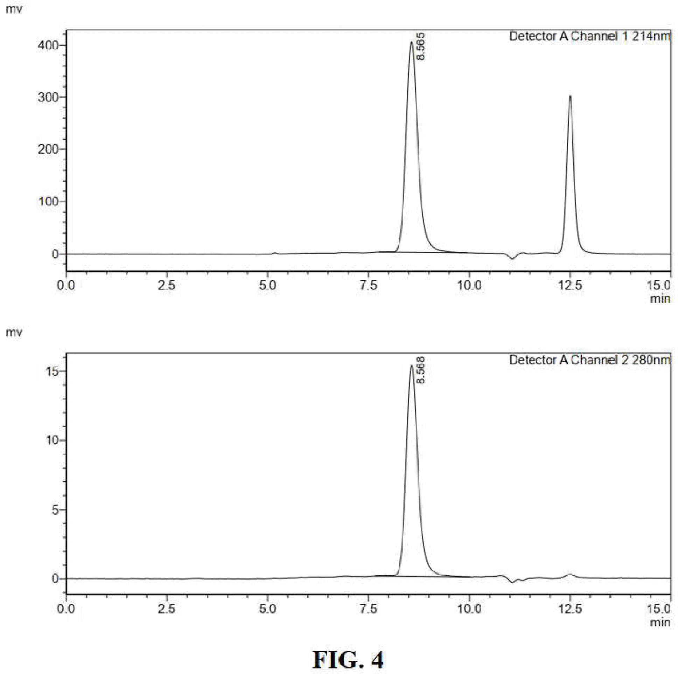

FIG. 4 shows the SEC-HPLC analysis results of H134 recombinant protein.

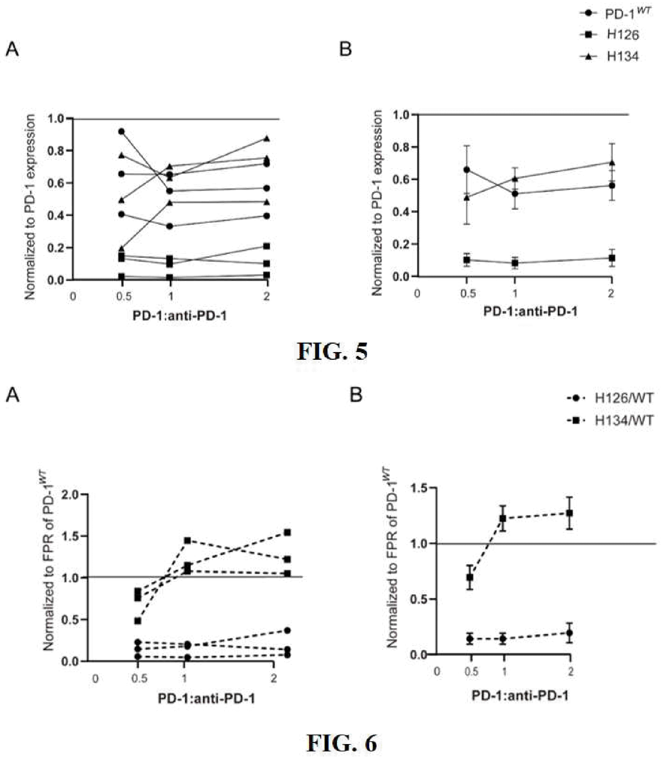

FIG. 5 illustrates the competitive binding of PD-1^{WT}, H126 recombinant protein, and H134 recombinant protein to pembrolizumab. A shows three replicate experiments of competitive binding to pembrolizumab by different concentrations of PD-1^{WT}, H126 recombinant protein and H134 recombinant protein against PD-1 expressed on T cells; and B shows the statistical analysis results of A.
FIG. 6 illustrates the recovery of free PD-1 (FPR) on T cells by H126 recombinant protein and H134 recombinant protein compared with PD-1^{WT}. A shows three replicate experiments of the blocking efficiency of different concentrations of H126 recombinant protein and H134 recombinant protein compared with PD-1^{WT}; and B shows the statistical analysis results of A.

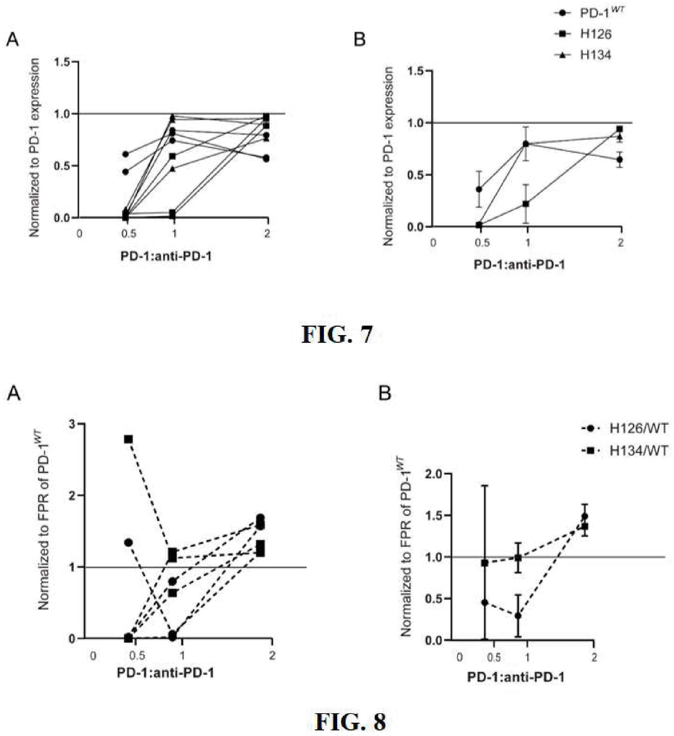

FIG. 7 illustrates the competitive binding of PD-1^{WT}, H126 recombinant protein, and H134 recombinant protein to nivolumab. A shows three replicate experiments of competitive binding to nivolumab by different concentrations of PD-1^{WT}, H126 recombinant protein and H134 recombinant protein against PD-1 expressed on T cells; and B shows the statistical analysis results of A.
FIG. 8 illustrates the recovery of free PD-1 (FPR) on T cells by H126 recombinant protein and H134 recombinant protein compared with PD-1^{WT}. A shows three replicate experiments of the blocking efficiency of different concentrations of H126 recombinant protein and H134 recombinant protein compared with PD-1^{WT}; and B shows the statistical analysis results of A.

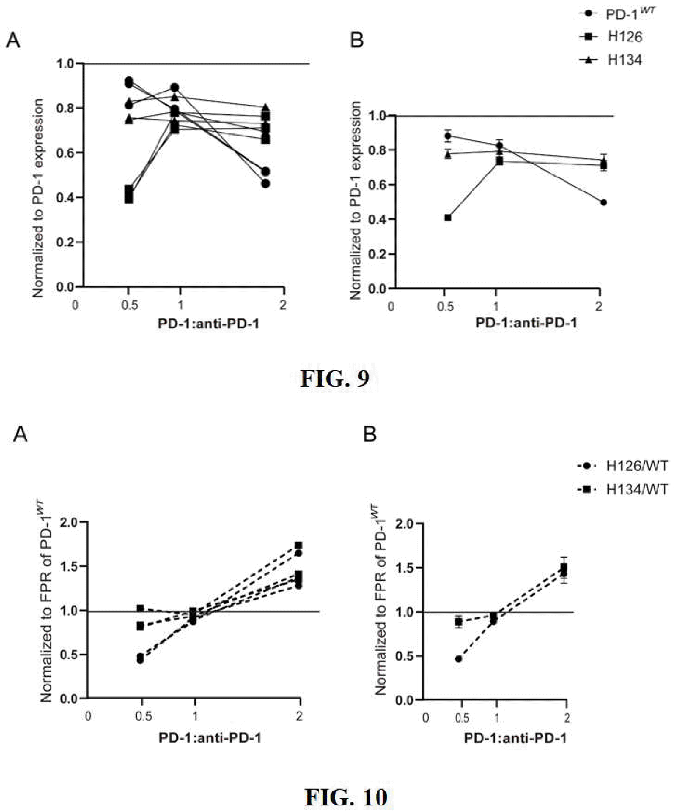

FIG. 9 illustrates the competitive binding of PD-1^{WT}, H126 recombinant protein, and H134 recombinant protein to sintilimab. A shows three replicate experiments of competitive binding to sintilimab by different concentrations of PD-1^{WT}, H126 recombinant protein and H134 recombinant protein against PD-1 expressed on T cells; and B shows the statistical analysis results of A.
FIG. 10 illustrates the recovery of free PD-1 (FPR) on T cells by H126 recombinant protein and H134 recombinant protein compared with PD-1^{WT}. A shows three replicate experiments of the blocking efficiency of different concentrations of H126 recombinant protein and H134 recombinant protein compared with PD-1^{WT}; and B shows the statistical analysis results of A.

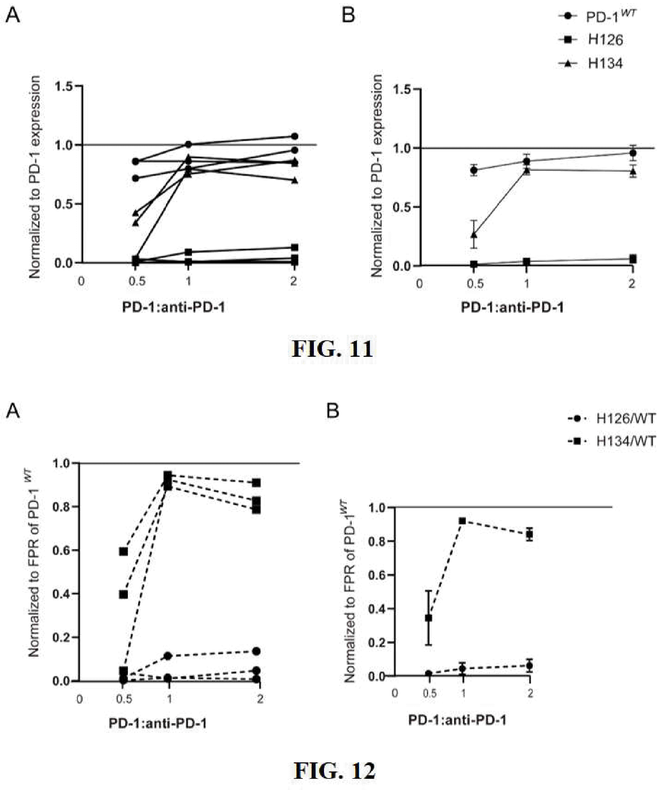

FIG. 11 illustrates the competitive binding of PD-1^{WT}, H126 recombinant protein, and H134 recombinant protein to tislelizumab. A shows three replicate experiments of competitive binding to tislelizumab by different concentrations of PD-1^{WT}, H126 recombinant protein and H134 recombinant protein against PD-1 expressed on T cells; and B shows the statistical analysis results of A.
FIG. 12 illustrates the recovery of free PD-1 (FPR) on T cells by H126 recombinant protein and H134 recombinant protein compared with PD-1^{WT}. A shows three replicate experiments of the blocking efficiency of different concentrations of H126 recombinant protein and H134 recombinant protein compared with PD-1^{WT}; and B shows the statistical analysis results of A.

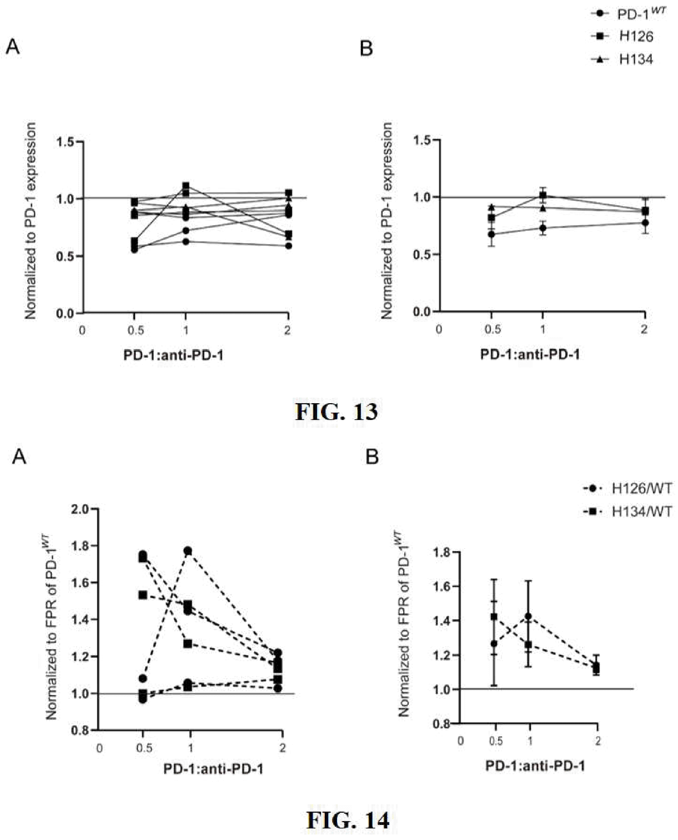

FIG. 13 illustrates the competitive binding of PD-1^{WT}, H126 recombinant protein, and H134 recombinant protein to toripalimab. A shows three replicate experiments of competitive binding to toripalimab by different concentrations of PD-1^{WT}, H126 recombinant protein and H134 recombinant protein against PD-1 expressed on T cells; and B shows the statistical analysis results of A.
FIG. 14 illustrates the recovery of free PD-1 (FPR) on T cells by H126 recombinant protein and H134 recombinant protein compared with PD-1^{WT}. A shows three replicate experiments of the blocking efficiency of different concentrations of H126 recombinant protein and H134 recombinant protein compared with PD-1^{WT}; and B shows the statistical analysis results of A.

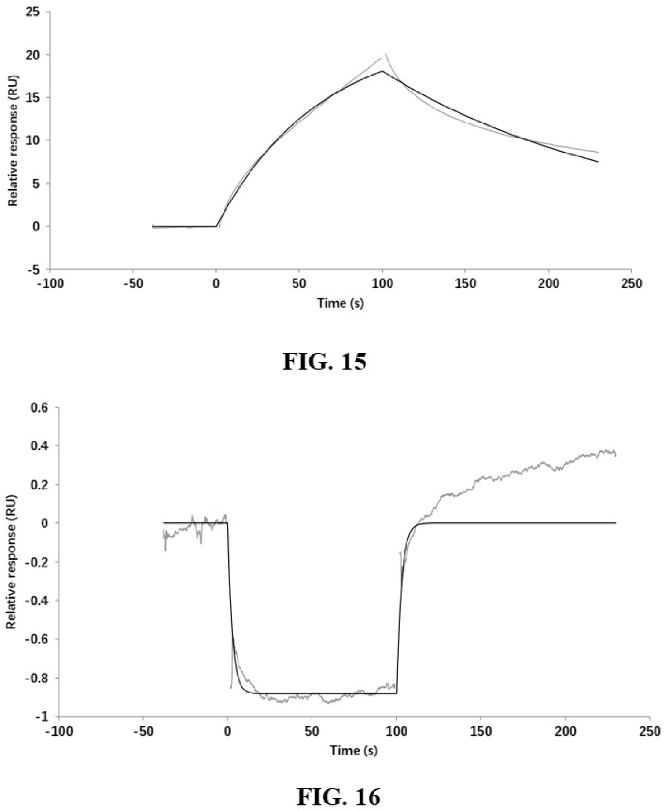

FIG. 15 shows the binding between PD-1^{WT} and PD-L1 detected by surface plasmon resonance technology.
FIG. 16 shows the binding between H126 recombinant protein and PD-L1 detected by surface plasmon resonance technology.

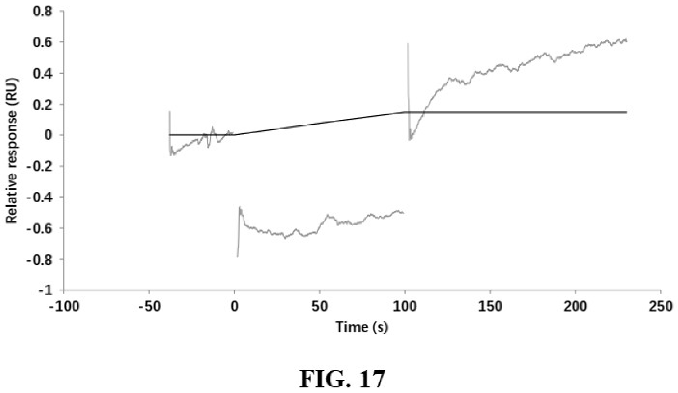

FIG. 17 shows the binding between H134 recombinant protein and PD-L1 detected by surface plasmon resonance technology.

### DETAILED DESCRIPTION

Unless otherwise defined, all technical and scientific terms used in the present invention have the same meaning as commonly used in the art to which the present invention belongs. For the purposes of interpreting this specification, the following definitions will apply, and where appropriate, terms used in the singular will also include the plural form, and vice versa.

Unless the context clearly indicates otherwise, the terms "a" and "an" as used herein include plural referents. For example, reference to "a cell" includes a plurality of such cells and equivalents known to those skilled in the art, and the like.

As used herein, a numerical range shall be construed as having recited every number within that range. For example, a range of 1 to 20 shall be understood to include any number, combination of numbers, or subrange from the group consisting of 1, 2, 3, 4, 5, 6, 7, 8, 9, 10, 11, 12, 13, 14, 15, 16, 17, 18, 19, or 20.

As used herein, the terms "comprises" or "comprising" mean "including, but not limited to". The term is intended to be open-ended to specify the presence of any of the stated features, elements, integers, steps, or components, but does not preclude the presence or addition of one or more other features, elements, integers, steps, components, or groups thereof. Accordingly, the term "comprising" includes the more restrictive terms "consisting of" and "substantially consisting of". In one embodiment, the term "comprising", as used throughout this application, particularly in the claims, may be replaced by the term "consisting of".

As used herein, the terms "optionally", "any", "arbitrary" or "any one" mean that the subsequently described event or circumstance may, but need not, occur, and the description includes instances where the event or circumstance occurs and instances where it does not. As used herein, the terms "a" and "an" are used to refer to one or more than one grammatical object in the present invention. As used herein, the term "and/or" shall be understood to mean any one of the optional items or a combination of any two or more of the optional items.

Experimental methods involved in the present invention:

### Flow cytometry:

The blood is diluted 1:1 with PBS, and the resulting dilution is then slowly layered onto the upper surface of the human lymphocyte separation medium in a 15 mL centrifuge tube at a ratio of 2:1;
Centrifugation is performed at room temperature at 2000 RPM with acceleration 1 and deceleration 1 for 22 min;
After centrifugation, the layers from top to bottom consist of a mixture of PBS and residual plasma, the buffy coat, lymphocyte separation medium, and blood cells. PBMCs reside in the middle buffy coat, which is then aspirated and transferred to another sterile centrifuge tube;
Sterile PBS in a volume at least three times that of the aspirated buffy coat is added, and centrifugation is carried out at 1500 RPM for 10 min at room temperature to remove residual lymphocyte separation medium, and the supernatant is discarded;
The pellet is resuspended in a small volume of PBS, adjusted to an appropriate volume with PBS, centrifuged again at 1200 RPM for 8 min to remove platelets and cell debris, and the cells are then resuspended in PBS to obtain a PBMC suspension;
Antigen (PD-1^{WT}, H126, H134) and antibody (pembrolizumab, nivolumab, sintilimab, tislelizumab, toripalimab) systems are prepared to final antigen concentrations of 2.5 µg/mL, 5 µg/mL, and 10 µg/mL, respectively, and a final antibody concentration of 5 µg/mL, yielding antigen: antibody ratios of 0.5:1, 1:1, and 2:1;
Antigens and commercial PD-1 antibodies at different ratios and concentrations are added to EP tubes, mixed by pipetting and incubated at 4°C for 30 min;
50 µL of PBMC suspension is taken, the above reaction mixture is added to the corresponding cell suspension, mixed, and incubated at 4°C for 30 min;
Cells are washed twice with 1 mL of PBS per tube and centrifuged at 300 g for 5 min;
The cells are resuspended in an appropriate volume of PBS, the ISO tubes are supplemented with flow antibodies PE-Cy7 anti-human CD3 and APC anti-human PD-1, the test tubes are supplemented with flow antibodies PE-Cy7 anti-human CD3 and APC anti-human PD-1, respectively, and the mixtures are vortexed and incubated at 4°C in the dark for 30 min;
2 mL of PBS is added, the mixture is centrifuged for 5 min, the supernatant is discarded, and the step is repeated once;
2 mL PBS is added, the mixture is centrifuged for 5 min, the supernatant is discarded, and the step is repeated once.

### Surface plasmon resonance technology:

It is performed using a Biacore X optical biosensor at 25°C. Human PD-L1 fusion protein is immobilized on the surface of a CM5 sensor chip via free amine coupling, followed by deactivation with ethanolamine. To determine the equilibrium dissociation constant (Kd), PD-1 or its mutants at concentrations ranging from 0.35 M to 20 M are sequentially injected into the flow cells at a rate of 20 L/min. The final response level is obtained by subtracting the background response of the reference flow cell from the total response of the experimental flow cell. Data are fitted to a nonlinear 1:1 Langmuir binding model to calculate Kd according to the equation: R RmaxC/(C Kd), where R is the response unit (RU) of bound analyte, Rmax is the maximum response level, C is the micromolar concentration of free analyte, and Kd is the equilibrium dissociation constant in micromolar. For rapid qualitative analysis of PD-1 mutants, equal amounts of the proteins at a concentration of 1.7 µM (below saturation level) are injected into the flow cells as well.

To make the objectives, technical solutions, and advantages of the present invention clearer, the present invention will be further described in detail below with reference to examples. For those examples in which specific conditions are not indicated, the experiments are carried out in accordance with conventional conditions or conditions recommended by the manufacturers. All reagents or instruments without indicated manufacturers are conventional commercially available products. To better illustrate the present invention, numerous specific details are set forth in the following detailed description. The specific embodiments described herein are for the purpose of explaining the present invention only and are not intended to constitute any limitation of the present invention. In addition, in the following description, descriptions of well-known structures and technologies are omitted to avoid unnecessarily obscuring the concept of the present invention. Such structures and techniques are also described in numerous publications, such as Molecular Cloning: A Laboratory Manual (Fourth Edition) (Cold Spring Harbor Laboratory Press), and Ausubel, F. M. et al., Current Protocols in Molecular Biology, published by Greene Publishing Assoc. and Wiley-Interscience.

### Example 1 Expression and purification of recombinant proteins

### 1.1 Construction of expression vectors

H126 recombinant protein: The nucleotide sequence as shown in SEQ ID NO: 4 was cloned into the expression vector pcDNA3.4 (with NotI/XbaI restriction sites). Positive clones were screened and sequenced with universal sequencing primers, and then an expression vector containing the coding sequence for the recombinant protein H126 was obtained.

H134 recombinant protein: The nucleotide sequence as shown in SEQ ID NO: 5 was cloned into the expression vector pcDNA3.4 (with NotI/XbaI restriction sites). Positive clones were screened and sequenced with universal sequencing primers, and then an expression vector containing the coding sequence for the recombinant protein H134 was obtained.

### 1.2 Recombinant protein expression, purification, and quality assessment

### (1) Cell transfection:

HEK293 cells at an appropriate confluency were harvested, and the supernatant was collected after centrifugation;
The cells were resuspended in approximately 0.5 mL of electroporation buffer, mixed gently, and then an appropriate amount of plasmid (the expression vector constructed above, at a concentration of 500 ng/µL) was added;
The above cell-plasmid suspension was mixed thoroughly, and 1 mL of it was transferred into a 1 mL electroporation cuvette; the electroporation cuvette was then placed in an electroporator for electroporation;
After electroporation, the cells in the cuvette were transferred into a pre-prepared shake flask containing 20 mL of culture medium and incubated statically for 40 min;
After incubation, the flask was placed in an incubator at 37°C, 270 rpm and 8% CO₂. Twenty-four hours later, feed medium/sodium butyrate/double antibiotics were added, and cultivation was continued for 3-7 days.

### (2) Purification by protein A affinity chromatography

The recombinant protein expressed in the above cultured cells was purified by affinity chromatography.
Column equilibration: 1×PBS, flow rate 1 mL/min, 20 mL
Sample loading: Flow rate 1 mL/min
Washing: 1×PBS, flow rate 1 mL/min, 20 mL
Elution: Citrate buffer (pH 3.4), 1 mL/min, collected in fractions of about 500 µL per tube. A total of 10 tubes were collected, and the absorbance at 280 nm was measured using a NanoDrop instrument.

### (3) Detection of recombinant protein purity

### 1) SDS PAGE detection

Preparation and assembly of the electrophoresis tank: The glass cassette with precast gel was taken out and placed into the electrode holder, with the short glass plate facing the red rubber strip of the electrode. The electrode holder was then inserted into the motor base and the switch was closed. The glass cassette with precast gel was taken out and placed into the electrode holder, with the short glass plate facing the red rubber strip of the electrode. The electrode holder was then inserted into the motor base and the switch was closed. The base was placed into the electrophoresis tank. Electrophoresis buffer was added into the center of the electrode holder to a level 1 cm above the stacking gel. Additional electrophoresis buffer was then added into the electrophoresis tank until the liquid level reached the top of the stacking gel.

Sample heating treatment: reducing heating at 99°C for 6 min; non-reducing heating at 99°C for 3 min.

Samples were loaded, subjected to gel electrophoresis, stained and destained, and photographed. The loading volume was 10 µL per well. Electrophoresis was performed at 180 V for 45 min. Staining and destaining were carried out for 15 min. Images were captured using a gel imaging system under white light illumination.

The SDS PAGE results are shown in FIG. 1 and FIG. 2.

### 2) SEC-HPLC detection

SEC analysis was performed using an LC-20AT high-performance liquid chromatography system coupled with a gel filtration column. The experimental conditions are listed in Table 1:

**Table 1**

| **Parameter** | **Value** |
|---|---|
| Flow rate | 1 mL/min |
| Injection volume | 20 µL |
| Column temperature | 35°C |
| Detection wavelength | 214 nm, 280 nm |
| Acquisition time | 15 min |

The water was replaced with the mobile phase, and the flow rate was gradually increased to 1.000 mL/min until the baseline became stable. 50 µL of the antibody was transferred into sample vials labeled with corresponding numbers, and the sample vials were placed into the corresponding positions of the instrument. The sampling time was set to 15 min. Data were analyzed, processed and saved. The mobile phase was then replaced with deionized water to flush the system for 1.5 h. The SEC-HPLC detection results are shown in FIG. 3 to FIG. 4.

### (4) Endotoxin detection

Preparation of sample positive control solution: A solution at twice the concentration of the test solution was mixed 1:1 with an endotoxin standard (0.24 EU/mL).

### Preparation of test solution:

Dilution factor of samples: MVD = C·L/λ, Where MVD is the maximum valid dilution factor of the test sample, L is the bacterial endotoxin limit of the test sample (1 EU/mg), C is the concentration of the test sample, and λ is the labeled sensitivity of the limulus amebocyte lysate.

The volume of each test solution is listed in Table 2.

**Table 2**

| Limulus amebocyte lysate (0.06 EU/mL) | Water for endotoxin test | Sample |
|---|---|---|
| Negative control tube | 200 µL | None |
| Positive control tube | 100 µL | 100 µL endotoxin standard (0.12 EU/mL) |
| Sample positive control tube | 100 µL | 100 µL positive control solution |
| Test sample tube | 100 µL | 100 µL test solution |

Sample detection: The tube openings were sealed, mixed gently, and incubated vertically in a constant-temperature incubator at 37°C for 60 min.

The detection results showed that the limulus amebocyte lysate remained clear and liquid without coagulation, indicating that the purified protein had a low endotoxin content.

### Example 2 Determination of the blocking effect of recombinant proteins on pembrolizumab (Keytruda)

The inventors define PD-1 on T cells that is not bound to anti-PD-1 antibodies as "free PD-1". After incubating T cells with anti-PD-1 antibody drugs, flow cytometry was performed to detect the expression of CD3, CD4, CD8 and PD-1 on peripheral blood T cells of tumor patients. If PD-1 molecules on T cells are bound by anti-PD-1 antibody drugs, they cannot be detected by fluorescently labeled anti-PD-1 flow cytometry antibodies. The expression level of PD-1 molecules on peripheral blood T cells before incubation with the anti-PD-1 antibody was used as the baseline. Free PD-1 recover (FPR) is defined as the recovery of free PD-1 to more than 50% of the baseline level.

Pembrolizumab is a monoclonal antibody that can bind to the PD-1 receptor, block the interaction between PD-1 and PD-L1 and PD-L2, relieve the suppression of the immune response mediated by the PD-1 pathway, and restore tumor-specific T-cell immunity.

In this example, flow cytometry was used to assess the ability of different concentrations of wild-type PD-1 recombinant protein (PD-1^{WT}, with its amino acid sequence as shown in SEQ ID NO: 1), H126 recombinant protein, and H134 recombinant protein to compete with endogenous PD-1 for binding to pembrolizumab.

The results showed that PD-1^{WT}, H126 recombinant protein, and H134 recombinant protein could all competitively bind to pembrolizumab against endogenous PD-1 molecules (FIG. 5). When PD-1 molecules on T cells are bound by anti-PD-1 antibody drugs, they cannot be detected by fluorescently labeled anti-PD-1 flow cytometry antibodies. Therefore, a higher level of detectable PD-1 molecules (i.e., a higher PD-1 expression level) indicates a stronger blocking effect (stronger competitive binding against endogenous PD-1 molecules). Statistical analysis of the detection results indicated that at a concentration ratio of 1:1-2 with pembrolizumab, H134 recombinant protein exhibited the best blocking effect on pembrolizumab, followed by PD-1^{WT}, and H126 recombinant protein was the last (FIG. 5).

The measurement of free PD-1 recovery (FPR) was also consistent with the above results. H134 recombinant protein had the optimal blocking effect on pembrolizumab, enabling free PD-1 recovery to reach over 50% of the baseline level, which was superior to H126 recombinant protein (FIG. 6).

### Example 3: Determination of the blocking effect of recombinant proteins on nivolumab (Opdivo)

Nivolumab binds to programmed death receptor 1 (PD-1), blocking its interaction with PD-L1 and PD-L2, thereby blocking PD-1 pathway-mediated immunosuppressive responses, including anti-tumor immune responses.

In this example, flow cytometry was performed to assess the ability of different concentrations of wild-type PD-1 recombinant protein (PD-1^{WT}), H126 recombinant protein and H134 recombinant protein to compete with endogenous PD-1 for binding to nivolumab.

The results showed that PD-1^{WT}, H126 recombinant protein and H134 recombinant protein all competitively bound to nivolumab against endogenous PD-1 molecules (FIG. 7). Statistical analysis of the detection results indicated that at a concentration ratio of 2:1 to nivolumab, H126 recombinant protein exhibited the best blocking effect on nivolumab, followed by H134 recombinant protein, and the PD-1^{WT} was the last (FIG. 7).

The measurement of free PD-1 recovery (FPR) was also consistent with the above results. At a concentration ratio of 2:1 to nivolumab, H126 recombinant protein had the optimal blocking effect on nivolumab, followed by H134 recombinant protein. Both recombinant proteins restored free PD-1 levels above baseline (FIG. 8).

### Example 4 Determination of the blocking effect of recombinant proteins on sintilimab

Sintilimab binds to the programmed death receptor 1 (PD-1) expressed on T cells, blocks its interaction with PD-L1 and PD-L2, and inhibits PD-1 pathway-mediated immunosuppressive responses, including antitumor immune responses.

In this example, flow cytometry was used to assess the ability of different concentrations of wild-type PD-1 recombinant protein (PD-1^{WT}), H126 recombinant protein and H134 recombinant protein to compete with endogenous PD-1 for binding to sintilimab.

The results showed that PD-1^{WT}, H126 recombinant protein and H134 recombinant protein all competitively bound to sintilimab against endogenous PD-1 molecules (FIG. 9). Statistical analysis of the detection results indicated that at a concentration ratio of 2: 1 to sintilimab, H126 recombinant protein and H134 recombinant protein exerted comparable blocking effects on sintilimab, both of which were superior to that of PD-1^{WT} (FIG. 9).

The measurement of free PD-1 recovery (FPR) was also consistent with the above results. At concentration ratios of 0.5-2:1 with sintilimab, both H126 recombinant protein and H134 recombinant protein restored free PD-1 to more than 50% of the baseline level (FIG. 10). At a concentration ratio of 2:1 to sintilimab, H126 recombinant protein and H134 recombinant protein exhibited equivalent effects, both recovering free PD-1 to above the baseline level.

### Example 5: Determination of the blocking effect of recombinant proteins on tislelizumab

Tislelizumab binds to the programmed death receptor 1 (PD-1) expressed on T cells, blocks its interaction with PD-L1 and PD-L2, and inhibits PD-1 pathway-mediated immunosuppressive responses, including antitumor immune responses.

In this example, flow cytometry was used to assess the ability of different concentrations of wild-type PD-1 recombinant protein (PD-1^{WT}), H126 recombinant protein and H134 recombinant protein to compete with endogenous PD-1 for binding to tislelizumab.

The results showed that PD-1^{WT}, H126 recombinant protein and H134 recombinant protein all competitively bound to tislelizumab against endogenous PD-1 molecules (FIG. 11). Statistical analysis of the detection results indicated that at concentration ratios of 0.5-2:1 with tislelizumab, PD-1^{WT} exhibited the best blocking effect, followed by H134 recombinant protein, and H126 recombinant protein was the last (FIG. 11).

The measurement of free PD-1 recovery (FPR) was also consistent with the above results. At concentration ratios of 1-2:1 with tislelizumab, H134 recombinant protein recovered free PD-1 to more than 50% of the baseline level, which was superior to H126 recombinant protein (FIG. 12).

### Example 6: Determination of the blocking effect of recombinant proteins on Treprinumab

Toripalimab binds to the programmed death receptor 1 (PD-1) expressed on T cells, blocks its interaction with PD-L1 and PD-L2, and inhibits PD-1 pathway-mediated immunosuppressive responses, including antitumor immune responses.

In this example, flow cytometry was used to assess the ability of different concentrations of wild-type PD-1 recombinant protein (PD-1^{WT}), H126 recombinant protein and H134 recombinant protein to compete with endogenous PD-1 for binding to toripalimab.

The results showed that PD-1^{WT}, H126 recombinant protein and H134 recombinant protein all competitively bound to toripalimab against endogenous PD-1 molecules (FIG. 13). At concentration ratios of 1-2:1 with toripalimab, H126 recombinant protein showed a better blocking effect, followed by H134 recombinant protein, and both recombinant proteins exhibited superior blocking effects to PD-1^{WT}.

The measurement of free PD-1 recovery (FPR) was also consistent with the above results. Both H126 recombinant protein and H134 recombinant protein recovered free PD-1 to above the baseline level (FIG. 14).

### Example 7: Recombinant proteins do not bind to the endogenous PD-L1

PD-L1 expressed on tumor cells binds to PD-1 expressed on T cells, delivering inhibitory signals to T cells and suppressing the anti-tumor activity of T cells. PD-1 antibody drugs block the binding between PD-1 expressed on T cells and PD-L1 expressed on tumor cells, thereby activating T cells. Since anti-PD-1 antibody drugs exert non-selective effects in vivo, they also cause the activation of T cells directed against self-antigens, leading to immune-related adverse events.

In this example, surface plasmon resonance technology was used to detect the binding of PD-1^{WT}, H126 recombinant protein, and H134 recombinant protein to PD-L1, respectively.

The results showed that PD-1^{WT} could bind to the endogenous PD-L1, whereas H126 recombinant protein and H134 recombinant protein did not bind to the endogenous PD-L1 (FIG. 15 to FIG. 17). Finally, it should be noted that the above contents are only used to illustrate the technical solutions of the present invention, rather than limiting the scope of protection of the present invention. Simple modifications or equivalent replacements to the technical solutions of the present invention by those of ordinary skill in the art shall not depart from the essence and scope of the technical solutions of the present invention.

## Claims

1. A protein molecule, wherein the protein molecule has 80%-100% sequence identity to a wild-type protein as shown in SEQ ID NO: 1;
preferably, the protein molecule is a functional variant that differs from the wild-type protein as shown in SEQ ID NO: 1 by at least one amino acid position;
the difference at amino acid positions is achieved by at least one of addition, deletion, modification and/or substitution of amino acids;
the functional variant has the same, similar, or better function in blocking the binding of anti-PD-1 antibodies to PD-1 molecules as the wild-type protein as shown in SEQ ID NO: 1.

2. The protein molecule according to claim 1, wherein the protein molecule comprises an amino acid substitution at position 126 or 134 compared to the wild-type protein as shown in SEQ ID NO: 1.

3. The protein molecule according to claim 2, wherein the protein molecule comprises an amino acid substitution of I126A compared to the wild-type protein as shown in SEQ ID NO: 1.

4. The protein molecule according to claim 3, wherein the protein molecule has the amino acid sequence as shown in SEQ ID NO: 2.

5. The protein molecule according to claim 2, wherein the protein molecule comprises the amino acid substitution of I134A compared to the wild-type protein as shown in SEQ ID NO: 1.

6. The protein molecule according to claim 5, wherein the protein molecule has the amino acid sequence as shown in SEQ ID NO: 3.

7. A recombinant protein, wherein the recombinant protein comprises the protein molecule according to any one of claims 1-6;
optionally, the recombinant protein further comprises a bioactive protein or a functional fragment thereof that assists in its expression and/or secretion, or prolongs its half-life in vivo.

8. An anti-PD-1 antibody blocker, wherein the blocker competes with endogenous PD-1 for bind to the anti-PD-1 antibody, does not bind to endogenous PD-L1, and does not block the binding of endogenous PD-L1 to endogenous PD-1, and the blocker comprises the protein molecule according to any one of claims 1-6 or the recombinant protein according to claim 7;
optionally, the blocker is formulated with a buffer.

9. Use of the protein molecule according to any one of claims 1-6, the recombinant protein according to claim 7, or the blocker of claim 8 in the preparation of a drug for treating immune-related adverse events.

10. Use of a protein molecule in the preparation of a drug for treating immune-related adverse events, wherein the protein molecule has the amino acid sequence as shown in SEQ ID NO: 1.
